# EUROPEAN PATENT SPECIFICATION

(11) **EP 4 306 504 B1**
(45) Date of publication and mention of the grant of the patent: **08.04.2026**
(21) Application number: 22788489.7
(22) Date of filing: 15.04.2022
(51) Int. Cl.: C07C 4/22, C07C 15/46, C07C 7/148, C07C 7/20, C08J 11/16, C08J 11/22, C08J 11/18

(54) **METHOD FOR RECOVERING STYRENE MONOMERS FROM WASTE POLYSTYRENE**
VERFAHREN ZUR RÜCKGEWINNUNG VON STYROLMONOMEREN AUS POLYSTYROLABFÄLLEN
PROCÉDÉ DE RÉCUPÉRATION DE MONOMÈRES DE STYRÈNE À PARTIR DE DÉCHETS DE POLYSTYRÈNE

(30) Priority: 16.04.2021 KR 20210049835; 04.03.2022 KR 20220028275
(43) Date of publication of application: 17.01.2024
(73) Proprietor: Korea Research Institute of Chemical Technology, Daejeon 34114 (KR)
(72) Inventor: YUN, Gwang Nam, Daejeon 34114 (KR); HWANG, Dong Won, Daejeon 34114 (KR); HWANG, Young Kyu, Daejeon 34114 (KR); HONG, Do Young, Daejeon 34114 (KR)
(74) Representative: Isarpatent
(86) International application number: PCT/KR2022/005448
(87) International publication number: WO 2022/220634

(56) References cited:
- JP-A- 2000 191 825
- JP-A- 2005 154 525
- JP-A- 2007 291 039
- KR-A- 20020 023 472
- KR-A- 20200 033 508
- A. KARADUMAN: "Pyrolysis of polystyrene plastic wastes with some organic compounds for enhancing styrene yield", ENERGY SOURCES, vol. 24, no. 7, July 2002 (2002-07-01), Taylor & Francis, London, GB, pages 667 - 674, XP093259793, ISSN: 0090-8312, DOI: 10.1080/00908310290086590
- J. ANWAR, ET AL.: "Catalytic depolymerisation of polystyrene", PROGRESS IN RUBBER, PLASTICS AND RECYCLING TECHNOLOGY, vol. 24, no. 1, 1 February 2008 (2008-02-01), Sage Publications, Thousand Oaks, CA, US, pages 47 - 51, XP093259726, ISSN: 1477-7606, DOI: 10.1177/147776060802400104

## Description

### Technical Field

The present disclosure relates to a method for recovering styrene monomer from waste polystyrene. More particularly, the present disclosure relates to a method for recovering styrene monomer from waste polystyrene by depolymerizing the waste polystyrene in the presence of a catalyst, characterized in that the side products produced by the side reaction are suppressed to recover a high yield and high purity of styrene monomer.

### Background Art

Along with industrial development, a large number of plastics are being used around the world, and Korea has become one of the world's top four plastic producers, producing more than 7 million tons of general-purpose plastic products last year. However, plastics are disposed of in large quantities after use, causing many environmental problems. Currently, waste plastics are mainly disposed of by landfills, but their long biodegradation time in the soil and the shortage of landfills cause serious environmental problems, so there is a lot of interest in developing technologies to recycle these waste plastics as resources.

Various methods have been proposed for the treatment of waste plastics, but reuse as fuel oil and raw materials with added value rather than simple physical addition or processing is considered the most desirable method in terms of environmental issues and economics. Recycling methods for waste plastics are classified into material recycling, which is the recycling of plastics in their original form or by processing, thermal recycling, such as incineration, and chemical recycling, which is the recovery of chemicals such as raw materials for resin.

In the case of physical recycling, waste polystyrenes are mainly recycled for the production of recycled resin, lightweight concrete, and adhesives, but their added value is very low as a physical recycling method, and waste plastics cannot be recycled after multiple physical recycling, resulting in a large amount of waste polystyrene. In addition, a large amount of contaminated waste polystyrene discharged from agricultural markets or construction waste is not cleaner than other waste polystyrene, making it difficult to use as a physical recycling method.

Furthermore, a large amount of contaminated waste polystyrene has a volume of about 50 times more than other waste polystyrene, making it difficult to physically recycle, so it is disposed of by landfill or incineration. However, the incineration method caused environmental problems such as dioxin generation.

Therefore, chemical regeneration methods are attracting attention, and the recovery of styrene, a monomer, from waste polystyrene was first attempted by Nishizaki et al. in 1997, and approximately 50% monomer recovery from polystyrene by pyrolysis at 733 K was reported. On the basis of these techniques, the effects of different catalysts have been investigated by many researchers, and many catalysts have been developed to increase the yield of styrene monomer.

As methods for recovering styrene monomer using the above catalysts, technologies for recovering styrene monomer using highly acidic metal oxides such as Co₃O₄, Fe₂O₃, Cr₂O₃, and CuO as catalysts (non-patent reference 0001), technologies for recovering styrene monomer using sulfate catalysts (patent references 0001 and 0002), and binary catalyst technologies prepared by carrying metal oxides as the main catalyst and basic catalysts as co-catalysts to silica and alumina (patent reference 0003) have been proposed. In addition it was found that the use of organic compounds, such as in Non-patent literature 1, during the thermal non-catalytic pyrolysis can increase the yield of styrene monomer.

However, when metal oxides such as Co₃O₄, Fe₂O₃, Cr₂O₃, and CuO, which are highly acidic, are used as catalysts, such as in Non-patent literature 2, carbocations are generated by cleavage of the single (C-C) bond of polystyrene, and the pyrolysis reaction proceeds with the chain separation of styrene, but since carbocations are unstable with a lack of two electrons, they cause side reactions such as attacking the surrounding benzene ring, which reduces the yield of styrene monomer.

On the other hand, when basic catalysts such as BaO, K₂O, MgO, ZnO, CaO (Non-patent literature 3), and sulfate catalysts were used, the pyrolysis reaction proceeded by satisfying the octet law to generate stable carboanions without lacking electrons and separating styrene continuously, but there was a problem that the selectivity of styrene monomer was reduced by increasing the by-products such as ethylbenzene and alpha-methylstyrene.

In particular, ethylbenzene has a similar boiling point to the end product, styrene monomer, which has a significant impact on economics due to the increased cost of separating commercially available high-purity (>99.6%) styrene monomer.

Therefore, new recovery methods are needed to recover the monomer styrene from waste polystyrene with high yield and high purity.

### [Related art Literature]

### [Patent Literatures]

(Patent literature 1) Japanese Patent Application Laid-open Publication No. 2001-294708 (published date: 2001. 10. 23)
(Patent literature 2) Korean Patent Application Laid-open Publication No. 2001-87093 (published date: 2001. 09. 15)
(Patent literature 3), Korean Patent Application Laid-open Publication No. 2003-0081717 (published date: 2003. 10. 22)

### [Non-patent literature]

(Non-patent literature 1) Energy Sources, Vol. 24, No. 7, 2002, 667
(Non-patent literature 2) Ind. Eng. Res., Vol. 34, No. 12, 1995, 4519
(Non-patent literature 3)Prog. Rubber Plast. Rec., Vol. 24, No. 1, 2008, 47

### Disclosure

### Technical Problem

The main objective of the present disclosure is to solve the above problems and to provide a method for recovering styrene monomer with high yield by suppressing the production of ethylbenzene, alpha-methyl styrene, benzene, toluene, etc., which are produced as side reactions in the conventional recovery process of waste polystyrene.

In particular, by suppressing the production of ethylbenzene, which has a similar boiling point to styrene monomer and is difficult to separate, the separation of styrene monomer from the depolymerization products of waste polystyrene is facilitated.

### Technical Solution

To achieve the above objectives, one embodiment of the present disclosure includes: (a) adding one or more solvents selected from tetrahydrofuran and methyl tetrahydrofuran and one or more depolymerization catalysts selected from potassium carbonate and potassium hydrogen carbonate to waste polystyrene to obtain a polystyrene-dissolved mixture; (b) distilling the polystyrene-dissolved mixture to separately recover the solvent; and (c) depolymerizing the solvent separately recovered mixture obtained in the step (b) above to obtain a styrene monomer-containing product, characterized in that the product obtained in the step (c) above has a weight ratio of ethylbenzene (EB) to styrene monomer (SM) (SM/EB) of at least 80, thereby providing a method for recovering styrene monomer from waste polystyrene.

In one preferred embodiment of the present disclosure, in the step (a) above, the tetrahydrofuran and/or methyl tetrahydrofuran may be characterized by adding from 100 parts by weight to 200 parts by weight of the waste polystyrene.

In a preferred embodiment of the present disclosure, the step (a) may be performed at room temperature and atmospheric pressure.

In a preferred embodiment of the present disclosure, the distillation in the step (b) may be performed at 80°C to 250°C.

In a preferred embodiment of the present disclosure, the depolymerization in the step (c) may be performed at 200°C to 600°C.

In one preferred embodiment of the present disclosure, the potassium carbonate and/or potassium hydrogen carbonate in the step (c) above may be characterized in that the potassium carbonate is present in an amount of from 1 part to 10 parts by weight per 100 parts by weight of the waste polystyrene.

In one preferred embodiment of the present disclosure, the solvents tetrahydrofuran and/or methyl tetrahydrofuran separated in the step (b) above may be characterized in that the solvent is reused in the dissolution of the waste polystyrene in the step (a) .

### Advantageous Effects

The method for recovering styrene monomer from waste polystyrene of the present disclosure depolymerizes the polystyrene with a combination of a specific solvent and a specific catalyst, thereby suppressing the production of ethylbenzene and the like as by-products in the recovery process of the styrene monomer, thereby increasing the yield of the recovered styrene monomer.

In addition, since the solvent used in the present disclosure is an eco-friendly solvent with a low boiling point, there is an effect of preventing environmental pollution caused by the use of toxic organic solvents, such as conventional toluene, while reducing the recovery cost for reusing the solvent.

### Description of Drawings

FIG. 1 is a schematic process diagram showing a method for recovering styrene monomer from waste polystyrene according to one embodiment of the present disclosure;
FIG. 2 is a graph showing the product yields generated in Examples 1 and 2 and Comparative Examples 1 through 6 according to the present disclosure;

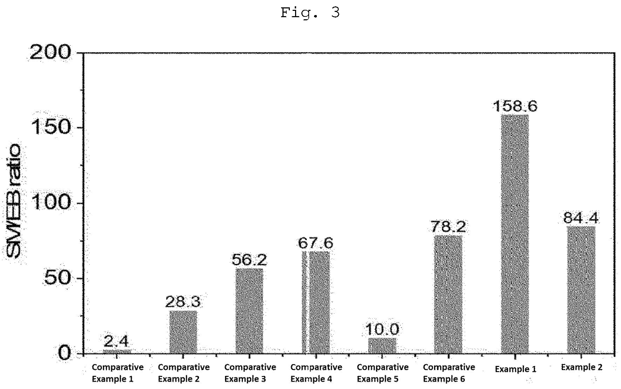

FIG. 3 is a graph showing the weight ratio of ethylbenzene to styrene monomer produced in Examples 1 and 2 and Comparative Examples 1 to 6 according to the present disclosure; and
FIG. 4 is a graph of XRD measurements of K₂CO₃ obtained after solvent evaporation from a mixture prepared by dissolving 5 g of K₂CO₃ and K₂CO₃ in the pristine state in 100 ml of THF and 100 ml of toluene, respectively.

### Mode for Invention

Unless defined otherwise, all technical and scientific terms used herein have the same meaning as commonly understood by those of ordinary skilled in the art to which this disclosure belongs. In general, the nomenclature used herein is well-known and commonly used in the art.

When a part of the present specification "includes" a component throughout the present specification, this means that it may further include other components rather than excluding the other component unless otherwise opposed.

The term "waste polystyrene" in this specification refers to polystyrene that has been discarded after being used in various industries, regardless of its shape or use.

*"Room temperature" in this specification refers to the temperature in a normal atmosphere, which may be 20°C ± 10°C, and "normal pressure" refers to the pressure in a normal atmosphere.

As used herein, terms such as "includes", "comprises", or "has" refer to the presence of, and refer to, a feature, number, step, operation, component, part, or combination thereof described in the specification. It does not exclude the possibility that other features, figures, steps, operations, components, parts, or combinations thereof may exist or may be added, unless otherwise noted.

The present disclosure relates to a method for recovering styrene monomer from waste polystyrene, the method including: (a) adding one or more solvents selected from tetrahydrofuran and methyl tetrahydrofuran and one or more depolymerization catalysts selected from potassium carbonate and potassium hydrogen carbonate to waste polystyrene to obtain a polystyrene-dissolved mixture; (b) distilling the polystyrene-dissolved mixture to separately recover the solvent; and (c) depolymerizing the solvent separately recovered mixture obtained in the step (b) above to obtain a styrene monomer-containing product, in which the product obtained in the step (c) above has a weight ratio of ethylbenzene (EB) to styrene monomer (SM) (SM/EB) of at least 80. In addition, the yield of styrene monomer by the method according to the present disclosure exceeds 70% by weight.

More specifically, the method for recovering styrene monomer, according to one embodiment of the present disclosure, depolymerizes polystyrene with a combination of a biomass-derived eco-friendly solvent, tetrahydrofuran and/or methyl tetrahydrofuran, and a depolymerization catalyst, potassium carbonate and/or potassium hydrogen carbonate, thereby suppressing the production of ethylbenzene and the like, which are produced as by-products in the recovery process of styrene monomer, thereby increasing the yield of recovered styrene monomer. At the same time, by dissolving the waste polystyrene by adding a depolymerization catalyst simultaneously with the solvent during the dissolution of the waste polystyrene, the depolymerization efficiency of the waste polystyrene can be increased due to the uniform dispersion of the depolymerization catalyst in the solvent phase.

Hereinafter, a preferred embodiment of a method for recovering styrene monomer from waste polystyrene according to the present disclosure will be described in detail with reference to the accompanying drawings.

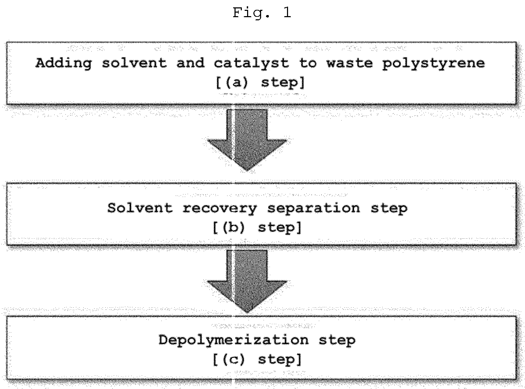

FIG. 1 is a schematic process diagram showing a method for recovering styrene monomer from waste polystyrene according to one embodiment of the present disclosure.

Referring to FIG. 1, a method for recovering styrene monomer from waste polystyrene according to one embodiment of the present disclosure includes first adding tetrahydrofuran and/or methyl tetrahydrofuran and potassium carbonate and/or potassium bicarbonate to the waste polystyrene at room temperature and under normal pressure, and stirring to obtain a mixture in which the polystyrene is dissolved [(step (a))].

In the present disclosure, tetrahydrofuran (THF), methyl tetrahydrofuran (MTHF), and mixtures thereof are used as solvents for dissolving waste polystyrene.

The tetrahydrofuran (THF) and methyl tetrahydrofuran (MTHF) have high solubility to polystyrene, while other synthetic resins such as polypropylene and polyethylene have low solubility, do not ignite, have a high vaporization point, and are made from biomass and are very eco-friendly. Therefore, the solvent can completely dissolve waste polystyrene within a short time at room temperature and atmospheric pressure without changing the properties of the polystyrene itself, thus improving the reduction efficiency.

Other solvents that can dissolve polystyrene include toluene, styrene, benzene, xylene, and limonene, but their solubility in polystyrene is low, and they are highly toxic to humans, so there may be problems from an environmental perspective. Their boiling point is almost the same as that of styrene monomer, so as the amount increases, the cost of separating the styrene monomer increases, which affects the economy.

In this disclosure, by dissolving waste polystyrene using tetrahydrofuran, methyl tetrahydrofuran, and mixtures thereof, the waste polystyrene can be completely dissolved within a short period of time even under room temperature and normal pressure to improve the dissolution efficiency, and the high vaporization point can prevent environmental pollution and loss of solvent due to natural evaporation even in the process of collecting solvent, as well as increase the ease of work processes such as solvent recovery described later.

The tetrahydrofuran and/or methyl tetrahydrofuran may be added in an amount of 100 parts by weight to 200 parts by weight per 100 parts by weight of waste polystyrene. When the amount of tetrahydrofuran and/or methyl tetrahydrofuran is less than 100 parts by weight of waste polystyrene, the dissolution of polystyrene may not be sufficient, and when the amount of tetrahydrofuran and/or methyl tetrahydrofuran exceeds 200 parts by weight, the recovery cost of the solvent may increase.

On the other hand, potassium carbonate (K₂CO₃) and/or potassium hydrogen carbonate (KHCO₃) are pyrolysis catalysts that catalyze pyrolysis reactions and are stable at high temperatures, resulting in high catalyst stability and easy recovery.

Furthermore, by adding the potassium carbonate (K₂CO₃) and/or potassium hydrogen carbonate (KHCO₃) to the polystyrene simultaneously with the tetrahydrofuran and/or methyl tetrahydrofuran at the time of dissolving the polystyrene, the catalyst can be prevented from agglomerating due to catalyst input in the conventional depolymerization step, and the efficient depolymerization of the polystyrene can be improved as catalysts are uniformly dispersed in the tetrahydrofuran and/or methyl tetrahydrofuran together with the polystyrene.

At this time, the potassium carbonate (K₂CO₃) and/or potassium hydrogen carbonate (KHCO₃) may be present in an amount of 1 to 10 parts by weight based on 100 parts by weight of the waste polystyrene. When the potassium carbonate (K₂CO₃) and/or potassium hydrogen carbonate (KHCO₃) is present in less than 1 part by weight per 100 parts by weight of waste polystyrene, the reactivity is greatly reduced, and the amount of ethylbenzene is greatly increased, and when the amount exceeds 10 parts by weight, the cost is increased without any difference in reactivity.

Such a mixture of tetrahydrofuran and/or methyl tetrahydrofuran with added potassium carbonate (K₂CO₃) and/or potassium hydrogen carbonate (KHCO₃) can be stirred at room temperature and normal pressure to dissolve the waste polystyrene. At this time, when the waste polystyrene is completely dissolved in the solvent, dissolved waste polystyrene can be suspended to precipitate foreign substances such as flame retardants, binders, coatings, etc., other than polystyrene contained in the waste polystyrene, and the precipitated foreign substances can be removed from the mixture by filtering.

On the other hand, the method for recovering styrene monomer, according to one embodiment of the present disclosure, may further include removing impurities such as plastic, paper, wood chips, food debris, small stones, etc., attached to the waste polystyrene by washing the waste polystyrene prior to step (a) above, and pulverizing the waste polystyrene to a size suitable for dissolution. At this time, the waste polystyrene to be pulverized may be pulverized to an average particle size of 1.0 cm to 10 cm, which is a size suitable for dissolution.

The polystyrene-dissolved mixture obtained by adding the tetrahydrofuran and/or methyl tetrahydrofuran with potassium carbonate (K₂CO₃) and/or potassium hydrogen carbonate (KHCO₃) is then distilled to separate and recover the solvent, tetrahydrofuran and/or methyl tetrahydrofuran, since the mixture may decompose at the reaction temperature or inhibit the catalytic reaction [step (b)].

The distillation may be performed at 80°C to 250°C under normal pressure using a conventional distiller. When distillation is performed at temperatures below 80°C, tetrahydrofuran and/or methyl tetrahydrofuran may not be properly separated and recovered from the mixture in which the polystyrene is dissolved, and when temperatures exceed 250°C, there may be problems with solvent recovery due to thermal decomposition of the solvent.

The separated and recovered tetrahydrofuran and/or methyl tetrahydrofuran can be reused for dissolving waste polystyrene in the step (a).

The mixture in which the polystyrene is dissolved, from which the tetrahydrofuran and/or methyl tetrahydrofuran have been separated, is then subjected to a depolymerization reaction in the presence of potassium carbonate (K₂CO₃) and/or potassium hydrogen carbonate (KHCO₃) to obtain a product containing styrene monomer [step (c)].

The depolymerization may be performed at 200°C to 600°C for 0.5 to 5 hours. When the depolymerization temperature is below 200°C, depolymerization may not occur, and reactivity may be greatly reduced, and when the depolymerization temperature exceeds 600°C, the amount of benzene and toluene may increase significantly due to the cracking reaction, resulting in a significant decrease in the amount of styrene monomer produced, and there may be problems with high boiling point dimers and trimers being mixed into the product. In addition, when the depolymerization time is less than 0.5 hours, the reaction time is not sufficient, and the recovery of styrene monomer is small. When the depolymerization time exceeds 5 hours, the amount of ethylbenzene increases greatly, and energy consumption increases, resulting in a decrease in productivity.

The product obtained from the depolymerization reaction can then be captured and cooled to recover the recovered product. At this time, the cooling method used in the art, such as an indirect cooling method or a direct cooling method in which the refrigerant is compressed and evaporated and the collected product is cooled using the evaporation heat of the refrigerant, may be used without limitation.

The products obtained from the above depolymerization reaction are not only styrene monomer (SM), which is the final target component, but also high-boiling substances such as ethylbenzene (EB), toluene, cumene, alpha methyl styrene, etc., and depolymerization reaction residues, which not only interfere with the depolymerization reaction, but also reduce the yield of styrene monomer, and conventionally, the yield of styrene monomer is reduced when operating for a long time.

Therefore, in the method of recovering styrene monomer according to the present disclosure, by dissolving and depolymerizing waste polystyrene using a potassium-containing carbonate catalyst with an eco-friendly solvent, a yield of styrene monomer, which is the final target component, can be recovered of more than 70%, and ethylbenzene (EB), toluene, cumene, and alpha methyl styrene can be recovered in a yield of 3.5% or less each. In particular, in a method for recovering styrene monomer according to the present disclosure, the styrene monomer can be recovered with a weight ratio of styrene monomer (SM) to ethylbenzene (EB) (SM/EB) of 80 or more, preferably 84 or more.

In other words, the method for recovering styrene monomer according to the present disclosure can inhibit the production of ethylbenzene, alpha-methyl styrene, toluene, and especially ethylbenzene, which are produced as side reactions in conventional styrene monomer recovery processes, thereby facilitating the separation process for recovering the final styrene monomer.

Hereinafter, the present disclosure will be described in more detail with reference to a specific example. The following examples are merely illustrative to help the understanding of the present disclosure, and the scope of the present disclosure is not limited thereto.

### [Example 1]

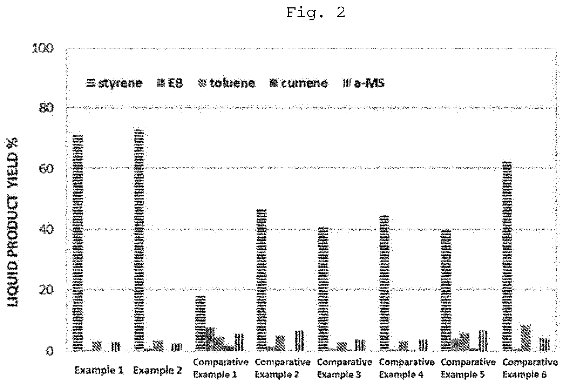

60 g of pulverized waste polystyrene was placed in a retractable reactor at room temperature, to which 100 g of tetrahydrofuran and 3 g of potassium carbonate (K₂CO₃) (Sigma-Aldrich, ACS reagent > 99%) as catalyst was added and stirred for 10 min at room temperature and atmospheric pressure to dissolve the waste polystyrene and disperse the catalyst. The mixture in which the waste polystyrene was dissolved was distilled at 200°C for 30 minutes to separate tetrahydrofuran. Thereafter, the temperature was raised to 375°C, and the depolymerization reaction was performed at this temperature for 1 hour. The product decomposed by the above depolymerization reaction was obtained by liquefaction in a condenser, and the obtained product was measured using GC/FID equipped with a capillary column (capillary column, HP-5, 30 m × 0.32 mm × 1.0 µm, crosslinked 5% PH ME siloxane), and the results are shown in Table 1, FIGS. 1 and 2.

### [Example 2]

The depolymerization of waste polystyrene was performed in the same way as in Example 1 to obtain the product, but the depolymerization was performed by adding 3 g of potassium hydrogen carbonate (KHCO₃) (Sigma-Aldrich, ACS reagent > 99.7%) instead of potassium carbonate (K₂CO₃) as a catalyst, and the product obtained was measured in the same way as in Example 1, and the results are shown in Table 1, FIGS. 1 and 2.

### [Comparative Example 1]

The depolymerization of waste polystyrene was performed in the same way as in Example 1 to obtain the product, but the depolymerization was performed without catalyst input, and the obtained product was measured in the same way as in Example 1, and the results are shown in Table 1, FIGS. 1 and 2.

### [Comparative Example 2]

The depolymerization of waste polystyrene was performed in the same way as in Example 1 to obtain the product, but the depolymerization was performed by adding 3 g of barium oxide (BaO) (Sigma-Aldrich reagent > 97%) instead of potassium carbonate (K₂CO₃) as a catalyst, and the obtained product was measured in the same way as in Example 1, and the results are shown in Table 1, FIGS. 1 and 2.

### [Comparative Example 3]

The depolymerization of waste polystyrene was performed in the same way as in Example 1 to obtain the product, but the depolymerization was performed by adding 3 g of calcium oxide (CaO) instead of potassium carbonate (K₂CO₃) as a catalyst, and the obtained product was measured in the same way as in Example 1, and the results are shown in Table 1, FIGS. 1 and 2.

### [Comparative Example 4]

The depolymerization of waste polystyrene was performed in the same way as in Example 1 to obtain the obtained product, but depolymerization was performed by adding 3 g of strontium oxide (SrO) instead of potassium carbonate (K₂CO₃) as a catalyst, and the obtained product was measured in the same way as in Example 1, and the results are shown in Table 1, FIGS. 1 and 2.

### [Comparative Example 5]

The depolymerization of waste polystyrene was performed in the same way as in Example 1 to obtain the obtained product, but depolymerization was performed by adding 3 g of KNO₃ instead of potassium carbonate (K₂CO₃) as a catalyst, and the obtained product was measured in the same way as in Example 1, and the results are shown in Table 1, FIGS. 1 and 2.

### [Comparative Example 6]

The depolymerization of waste polystyrene was performed in the same way as in Example 1 to obtain the product, but depolymerization was performed using toluene instead of tetrahydrofuran as a solvent, and the obtained product was measured in the same way as in Example 1, and the results are shown in Table 1, FIGS. 1 and 2.

**[Table 1]**

| Division | Catalyst | Solvent | Product yield(wt%) | | | | | | SM/EB Parts by weight |
|---|---|---|---|---|---|---|---|---|---|
| | | | SM | EB | TOL | CUM | AMS | Total liquid | |
| Example 1 | K₂CO₃ | THF | 71.067 | 0.448 | 3.220 | 0.000 | 2.943 | 77.678 | 158.6 |
| Example 2 | KCHO₃ | THF | 72.628 | 0.861 | 3.410 | 0.000 | 2.458 | 79.357 | 84.4 |
| Comparative Example 1 | - | THF | 18.539 | 7.744 | 4.656 | 1.732 | 5.661 | 38.333 | 2.4 |
| Comparative Example 2 | BaO | THF | 46.698 | 1.653 | 4.943 | 0.271 | 6.717 | 60.283 | 28.3 |
| Comparative Example 3 | CaO | THF | 41.153 | 0.732 | 2.858 | 0.081 | 3.868 | 48.695 | 56.2 |
| Comparative Example 4 | SrO | THF | 44.797 | 0.663 | 3.305 | 0.059 | 3.714 | 52.538 | 67.6 |
| Comparative Example 5 | KNO₃ | THF | 39.523 | 3.943 | 5.714 | 0.782 | 6.747 | 56.709 | 10.0 |
| Comparative Example 6 | K₂CO₃ | TOL | 62.352 | 0.797 | 8.453 | 0.000 | 4.357 | 75.958 | 78.2 |
| THF: Tetrahydrofuran, TOL: Toluene, SM: styrene monomer, EB: Ethylbenzene, CUM: Cumene, AMS: alphamethylstyrene | | | | | | | | | |

As shown in Table 1, FIGS. 1 and 2, Examples 1 and 2 showed higher yields of styrene monomer (SM) compared to Comparative Examples 1 through 6, while yields of the side reaction products ethylbenzene (EB), toluene, cumene, and alpha methyl styrene (a-MS) were lower. In particular, in Examples 1 and 2, the yield of styrene monomer (SM) was 70% by weight or more, and the weight ratio of styrene monomer (SM) to ethylbenzene (EB) was 84 or more.

When comparing Examples 1 and 2 with Comparative Examples 1 to 5, it was found that the presence or absence of a catalyst has a great relationship with the yield of styrene monomer (SM) and the amount of side reaction products generated. In particular, even when the same potassium salt was used, there was a significant difference in activity depending on the type of anion, with carbonate (CO₃²⁻) or bicarbonate (HCO₃⁻) resulting in a higher yield of styrene monomer (SM) and lower by-products such as ethylbenzene (EB).

In addition, when comparing the results of Example 1 and Comparative Example 6, even when the type of catalyst is the same, the type of solvent that dissolves waste styrene is also related to the yield of the final styrene monomer and the amount of side reaction products generated. Comparative Example 6 uses toluene as a solvent, which is conventionally used as a solvent to reduce waste styrene, and Example 1 uses THF as a solvent. When toluene is used as a solvent, the yield of styrene monomer is 62.3% by weight, which is only about 88% by weight when THF is used as a solvent, and the yield of ethylbenzene is high.

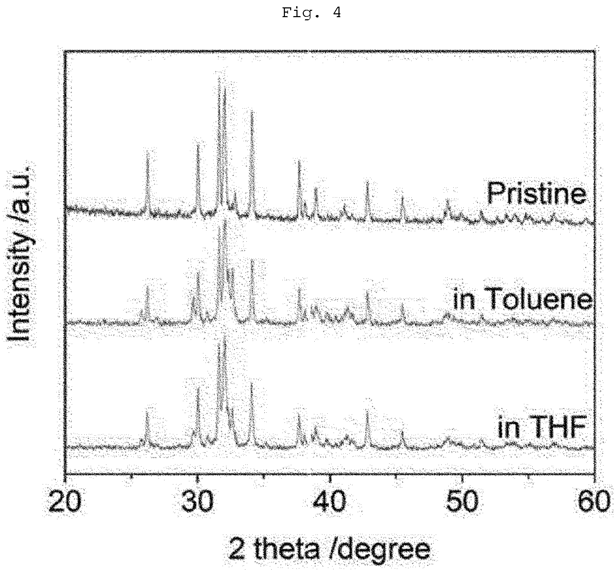

To determine the cause of this difference in activity, a mixture prepared by dissolving 5 g of K₂CO₃ and K₂CO₃ in the pristine state in 100 ml of THF and 100 ml of toluene, each, was stirred for 24 hours, and then the solvent was evaporated at 120°C for 12 hours, and the obtained K₂CO₃ was analyzed by XRD (Rigaku Ultima IV), and the results are shown in FIG. 4.

Referring to FIG. 4, the K₂CO₃ after dissolution in THF and toluene shows an overall change in crystallinity compared to the K₂CO₃ in the pristine state, but especially in the case of toluene, the peak at 2 theta = 32.5 degrees is significantly increased and the peak at 2 theta = 31.6 degrees in the case of toluene and a decrease in the peak at 2 theta = 31.5 degrees in the case of THF, indicating that the K₂CO₃ structure is more stable in THF solution than in toluene and less tendency to phase transformation.

Thus, when depolymerizing waste polystyrene with a combination of solvent and catalyst, such as in the present disclosure, in which one or more of THF and MTHF are selected as the solvent, and one or more of K2CO3 and KHCO3 are selected as the depolymerization catalyst, styrene monomer can be recovered at a high yield by depolymerization, so the production of ethylbenzene, which has a boiling point similar to that of styrene monomer, can be suppressed, thereby reducing the cost of the product separation process, and thus greatly improving the efficiency of the depolymerization process of waste polystyrene in practical industrial aspects.

While the above describes preferred embodiments of the disclosure, the disclosure is not limited thereto, and various modifications are possible and within the scope of the patent claims, the detailed description of the disclosure, and the accompanying drawings.

## Claims

1. A method for recovering styrene monomer from waste polystyrene, the method comprising:
(a) obtaining a polystyrene-dissolved mixture by adding at least one solvent selected from among tetrahydrofuran and methyl tetrahydrofuran and at least one depolymerization catalyst selected from among potassium carbonate and potassium hydrogen carbonate to waste polystyrene;
(b) separating and recovering the solvent by distilling the polystyrene-dissolved mixture; and
(c) depolymerizing the mixture recovered through solvent separation obtained in the step (b) to obtain a product containing a styrene monomer,
wherein
in the product obtained in the step (c), a weight ratio (SM/EB) of ethylbenzene (EB) to styrene monomer (SM) is 80 or more.

2. The method of claim 1, wherein in the step (a), the tetrahydrofuran and/or the methyl tetrahydrofuran are added in an amount of 100 to 200 parts by weight per 100 parts by weight of the waste polystyrene.

3. The method of claim 1, wherein the step (a) is performed at room temperature and atmospheric pressure.

4. The method of claim 1, wherein the distillation in the step (b) is performed at 80°C to 250°C.

5. The method of claim 1, wherein the depolymerization in the step (c) is performed at 200°C to 600°C.

6. The method of claim 1, wherein in the step (c), the tetrahydrofuran and/or the methyl tetrahydrofuran are present in an amount of 1 to 10 parts by weight per 100 parts by weight of the waste polystyrene.

7. The method of claim 1, wherein the solvent separated in the step (b) is reused for dissolving the waste polystyrene in the step (a).

## Patentansprüche

1. Verfahren zur Rückgewinnung von Styrolmonomer aus Polystyrolabfällen, wobei das Verfahren umfasst:
(a) Erhalten einer Polystyrol-gelösten Mischung durch Zugabe mindestens eines Lösungsmittels, ausgewählt aus Tetrahydrofuran und Methyltetrahydrofuran, und mindestens eines Depolymerisationskatalysators, ausgewählt aus Kaliumcarbonat und Kaliumhydrogencarbonat, zu Polystyrolabfällen;
(b) Trennen und Gewinnen des Lösungsmittels durch Destillieren der mit Polystyrol gelösten Mischung; und
(c) Depolymerisieren der in Schritt (b) durch Lösungsmittelabtrennung zurückgewonnenen Mischung, um ein Produkt zu erhalten, das Styrolmonomer enthält,
wobei
in dem in Schritt (c) erhaltenen Produkt das Gewichtsverhältnis (SM/EB) von Ethylbenzol (EB) zu Styrolmonomer (SM) 80 oder mehr beträgt.

2. Verfahren nach Anspruch 1, wobei in Schritt (a) das Tetrahydrofuran und/oder das Methyltetrahydrofuran in einer Menge von 100 bis 200 Gewichtsteilen pro 100 Gewichtsteilen des Polystyrolabfalls zugegeben werden.

3. Verfahren nach Anspruch 1, wobei der Schritt (a) bei Raumtemperatur und Atmosphärendruck durchgeführt wird.

4. Verfahren nach Anspruch 1, wobei die Destillation in Schritt (b) bei 80 °C bis 250 °C durchgeführt wird.

5. Verfahren nach Anspruch 1, wobei die Depolymerisation in Schritt (c) bei 200 °C bis 600 °C durchgeführt wird.

6. Verfahren nach Anspruch 1, wobei in Schritt (c) das Tetrahydrofuran und/oder das Methyltetrahydrofuran in einer Menge von 1 bis 10 Gewichtsteilen pro 100 Gewichtsteile des Polystyrolabfalls vorhanden sind.

7. Verfahren nach Anspruch 1, wobei das in Schritt (b) abgetrennte Lösungsmittel zum Auflösen des Polystyrolabfalls in Schritt (a) wiederverwendet wird.

## Revendications

1. Procédé de récupération du monomère styrène à partir de déchets de polystyrène, le procédé comprenant :
(a) l'obtention d'un mélange dissous dans du polystyrène en ajoutant au moins un solvant choisi parmi le tétrahydrofurane et le méthyl-tétrahydrofurane et au moins un catalyseur de dépolymérisation choisi parmi le carbonate de potassium et l'hydrogénocarbonate de potassium au polystyrène usagé ;
(b) la séparation et la récupération du solvant par distillation du mélange dissous dans le polystyrène ; et
(c) la dépolymérisation du mélange récupéré par séparation du solvant obtenue à l'étape (b) pour obtenir un produit contenant un monomère styrène,
dans lequel
dans le produit obtenu à l'étape (c), le rapport pondéral (SM/EB) entre l'éthylbenzène (EB) et le monomère styrène (SM) est supérieur ou égal à 80.

2. Procédé selon la revendication 1, dans lequel, à l'étape (a), le tétrahydrofurane et/ou le méthyl tétrahydrofurane sont ajoutés en une quantité de 100 à 200 parties en poids pour 100 parties en poids de polystyrene usagé.

3. Procédé selon la revendication 1, dans lequel l'étape (a) est réalisée d' e à température ambiante et à pression atmosphérique.

4. Procédé selon la revendication 1, dans lequel la distillation de l'étape (b) est effectuée à une température comprise entre 80 °C et 250 °C.

5. Procédé selon la revendication 1, dans lequel la dépolymérisation de l'étape (c) est réalisée à une température comprise entre 200 °C et 600 °C.

6. Procédé selon la revendication 1, dans lequel, à l'étape (c), le tétrahydrofurane et/ou le méthyl tétrahydrofurane sont présents en une quantité de 1 à 10 parties en poids pour 100 parties en poids de polystyrène usagé.

7. Procédé selon la revendication 1, dans lequel le solvant séparé à l'étape (b) est réutilisé pour dissoudre le polystyrène usagé à l'étape (a).
